(19) Europäisches Patentamt
European Patent Office
Office européen des brevets

(11) **EP 4 406 638 A1**

(12) **EUROPEAN PATENT APPLICATION**
published in accordance with Art. 153(4) EPC

(43) Date of publication:
**31.07.2024  Bulletin 2024/31**

(21) Application number: **22880828.3**

(22) Date of filing: **03.10.2022**

(51) International Patent Classification (IPC):
**B01D 53/047** (2006.01)     **F04B 27/02** (2006.01)

(52) Cooperative Patent Classification (CPC):
**B01D 53/047; F04B 27/02**

(86) International application number:
**PCT/JP2022/036902**

(87) International publication number:
**WO 2023/063132 (20.04.2023 Gazette 2023/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **14.10.2021   JP 2021168783**

(71) Applicant: **Daikin Industries, Ltd.
Osaka-shi, Osaka 530-0001 (JP)**

(72) Inventors:
• **IKEMIYA Makoto
  Osaka-shi, Osaka 530-0001 (JP)**
• **KAMEI Noritaka
  Osaka-shi, Osaka 530-0001 (JP)**
• **NAKATANI Hiroyuki
  Osaka-shi, Osaka 530-0001 (JP)**
• **KONDO Keita
  Osaka-shi, Osaka 530-0001 (JP)**

(74) Representative: **Hoffmann Eitle
Patent- und Rechtsanwälte PartmbB
Arabellastraße 30
81925 München (DE)**

(54)    **GAS COMPOSITION ADJUSTMENT DEVICE**

(57)    A gas composition adjustment apparatus includes an adsorber, a supply pump (310), and a discharge pump (320). The gas composition adjustment apparatus performs a pressurization operation in which the supply pump (310) supplies a gas to the adsorber; and a depressurization operation in which the discharge pump (320) discharges a gas from the adsorber. Each of the supply pump (310) and the discharge pump (320) is a positive displacement pump and includes a sealing member (313, 323). The sealing member (323) of the discharge pump (320) contains a fluororesin and a fibrous carbon material as main components.

FIG.8

## Description

### TECHNICAL FIELD

The present disclosure relates to a gas composition adjustment apparatus.

### BACKGROUND ART

Patent Document 1 discloses an oxygen concentrator. This oxygen concentrator is a gas composition adjustment apparatus of what is called a pressure swing adsorption (PSA) type.

The oxygen concentrator of Patent Document 1 includes an adsorption vessel filled with an adsorbent. This oxygen concentrator performs a pressurization operation in which a supply pump supplies air (atmospheric air) to the adsorption vessel so that the adsorbent adsorbs nitrogen in the air, and a depressurization operation in which a discharge pump sucks gas from the adsorption vessel so that the adsorbent desorbs nitrogen.

In the pressurization operation, the adsorbent in the adsorption vessel adsorbs nitrogen in the air, and oxygen-enriched gas flows out from the adsorption vessel. The oxygen-enriched gas has an oxygen concentration higher than that of the atmospheric air and a nitrogen concentration lower than that of the atmospheric air. In the depressurization operation, nitrogen-enriched gas, which contains nitrogen as a main component desorbed from the adsorbent in the adsorption vessel, is sucked from the adsorption vessel by the discharge pump. The nitrogen-enriched gas has a nitrogen concentration higher than that of the atmospheric air and an oxygen concentration lower than that of the atmospheric air.

The oxygen concentrator of Patent Document 1 includes a single air pump comprised of a supply pump and a discharge pump integrated together. Each of the supply pump and the discharge pump is a positive displacement pump including a cylinder, a piston, and a sealing member. The sealing member is attached to the piston and seals a gap between the cylinder and the piston.

### CITATION LIST

### PATENT DOCUMENT

PATENT DOCUMENT 1: WO 2013/042557

### SUMMARY OF THE INVENTION

### TECHNICAL PROBLEMS

In a gas composition adjustment apparatus including a positive displacement pump, a sealing member attached to a piston slides on a cylinder. Thus, the sealing member wears gradually. When the sealing member has worn to some extent, more gas leaks from the gap between the cylinder and the piston. As a result, oxygen-enriched gas and nitrogen gas are less produced, and the gas composition adjustment apparatus produces a lower performance.

It is an object of the present disclosure to provide a sealing member having a longer life and a gas composition adjustment apparatus having greater reliability.

### SOLUTION TO THE PROBLEMS

A first aspect of the present disclosure is a gas composition adjustment apparatus (100) including: an adsorber (234, 235) containing an adsorbent that adsorbs at least one of components of a gas to be treated; a supply pump (310) configured to supply the gas to be treated to the adsorber (234, 235); and a discharge pump (320) configured to suck a gas from the adsorber (234, 235). The gas composition adjustment apparatus (100) is configured to perform a pressurization operation in which the supply pump (310) pressurizes the gas to be treated and supplies the gas pressurized to the adsorber (234, 235), and the gas flowing out from the adsorber (234, 235) is sent out as a first gas; and a depressurization operation in which the discharge pump (320) sucks a gas from the adsorber (234, 235) and discharges the sucked gas, and the gas discharged from the discharge pump (320) is sent out as a second gas. Each of the supply pump (310) and the discharge pump (320) is a positive displacement pump including a cylinder (311, 321); a piston (312, 322) housed in the cylinder (311, 321); and a sealing member (313, 323) attached to the piston (312, 322) to seal a gap between the cylinder (311, 321) and the piston (312, 322). The sealing member (323) of the discharge pump (320) contains a fluororesin and a fibrous carbon material as main components.

In the discharge pump (320) of the first aspect, when the sealing member (323) slides on the cylinder (321), the fibrous carbon material is exposed on the surface of the sealing member (323) and comes into contact with the cylinder (321). The fibrous carbon material has an elongated shape, and thus is unlikely to fall off from the sealing member (323) when coming into contact with the cylinder (321). The sealing member (323) containing a fibrous carbon material as a main component is relatively unlikely to wear because the wear reduction effect of a fibrous carbon material lasts for a long period of time. Thus, in this aspect, the sealing member (323) of the discharge pump (320) has a longer life, and the gas composition adjustment apparatus (100) has greater reliability.

A second aspect of the present disclosure is an embodiment of the first aspect. In the second aspect, the sealing member (313) of the supply pump (310) contains a fluororesin and a particulate carbon material as main components.

In the second aspect, the supply pump (310) includes the sealing member (313) containing a fluororesin and a particulate carbon material as main components.

A third aspect of the present disclosure is an embodiment of the first or second aspect. In the third aspect, the sealing member (313) of the supply pump (310) has a linear expansion coefficient smaller than a linear expansion coefficient of the sealing member (323) of the discharge pump (320).

As the sealing member (313, 323) more expands thermally, the sealing member (313, 323) is pressed onto the cylinder (311, 321) with a greater force, and thus the sealing member (313, 323) is worn more. The supply pump (310) generally has a temperature higher than that of the discharge pump (320).

In the third aspect, the sealing member (313) of the supply pump (310) has a linear expansion coefficient smaller than a linear expansion coefficient of the sealing member (323) of the discharge pump (320). Thus, as compared to the sealing member (313) of the supply pump (310) having the same linear expansion coefficient as that of the sealing member (323) of the discharge pump (320), the sealing member (313) less expands thermally in the supply pump (310) whose temperature becomes higher than that of the discharge pump (320). Thus, in the supply pump (310) of this aspect, the sealing member (313) is pressed onto the cylinder (311) with a smaller force, and the sealing member (313) has a longer life.

A fourth aspect of the present disclosure is an embodiment of any one of the first to third aspects. In the fourth aspect, the sealing member (313) of the supply pump (310) has a Young's modulus higher than a Young's modulus of the sealing member (323) of the discharge pump (320).

As the sealing member (313, 323) is deformed more due to application of a gas pressure, the sealing member (313, 323) is pressed onto the cylinder (311, 321) with a greater force, and thus the sealing member (313, 323) is worn more. A gas pressure applied on the sealing member (313) of the supply pump (310) is generally higher than a gas pressure applied on the sealing member (323) of the discharge pump (320).

In the fourth aspect, the sealing member (313) of the supply pump (310) has a Young's modulus higher than a Young's modulus of the sealing member (323) of the discharge pump (320). Thus, as compared to the sealing member (313) of the supply pump (310) having the same Young's modulus as that of the sealing member (323) of the discharge pump (320), the sealing member (313) is less deformed by application of a gas pressure in the supply pump (310). Thus, in the supply pump (310) of this aspect, the sealing member (313) is pressed onto the cylinder (311) with a smaller force, and the sealing member (313) has a longer life.

A fifth aspect of the present disclosure is an embodiment of any one of the first to fourth aspects. In the fifth aspect, the gas to be treated is atmospheric air, the adsorbent in the adsorber (234, 235) adsorbs nitrogen which is a component of the gas to be treated, the first gas has a lower nitrogen concentration and a higher oxygen concentration as compared to the gas to be treated, the second gas has a higher nitrogen concentration and a lower oxygen concentration as compared to the gas to be treated, and an operation is performed to supply the second gas to a storage (2) for storing a fresh product.

In the fifth aspect, the gas composition adjustment apparatus (100) produces the first gas and the second gas by treating atmospheric air as a gas to be treated. The gas composition adjustment apparatus (100) of this aspect supplies, to the storage (2) for storing a fresh product, the second gas having a higher nitrogen concentration and a lower oxygen concentration as compared to the atmospheric air.

A sixth aspect of the present disclosure is an embodiment of any one of the first to fourth aspects. In the sixth aspect, the gas to be treated is atmospheric air, the adsorbent in the adsorber adsorbs nitrogen which is a component of the gas to be treated, the first gas has a lower nitrogen concentration and a higher oxygen concentration as compared to the gas to be treated, the second gas has a higher nitrogen concentration and a lower oxygen concentration as compared to the gas to be treated, and an operation is performed to supply the first gas to a user.

In the sixth aspect, the gas composition adjustment apparatus (100) produces the first gas and the second gas by treating atmospheric air as a gas to be treated. The gas composition adjustment apparatus (100) of this aspect supplies, to the user, the first gas having a lower nitrogen concentration and a higher oxygen concentration as compared to the atmospheric air.

BRIEF DESCRIPTION OF THE DRAWINGS

FIG. 1 is a perspective view of a transportation refrigeration apparatus including a gas composition adjustment apparatus according to a first embodiment.
FIG. 2 is a schematic vertical sectional view of the transportation refrigeration apparatus including the gas composition adjustment apparatus, and a transportation container including the transportation refrigeration apparatus, according to the first embodiment.
FIG. 3 is a piping system diagram showing a configuration of the gas composition adjustment apparatus according to the first embodiment.
FIG. 4 corresponds to FIG. 3 and shows the gas composition adjustment apparatus performing a first operation.
FIG. 5 corresponds to FIG. 3 and shows the gas composition adjustment apparatus performing a second operation.
FIG. 6 is a perspective view of an air pump of the gas composition adjustment apparatus.

FIG. 7 is a plan view of the air pump of the gas composition adjustment apparatus.

FIG. 8 is an exploded perspective view of the air pump of the gas composition adjustment apparatus.

FIG. 9 is a schematic cross-sectional view of a supply pump of the air pump.

FIG. 10 is a schematic cross-sectional view of a discharge pump of the air pump.

FIG. 11 is a cross-sectional view schematically showing a contact portion between an air seal and a cylinder.

FIG. 12 is a graph showing temporal changes in a suction-side pressure and a discharge-side pressure in each of the supply pump and the discharge pump.

FIG. 13 is a piping system diagram showing a configuration of a gas composition adjustment apparatus according to a second embodiment.

DESCRIPTION OF EMBODIMENTS

Embodiments of the present disclosure will be described below with reference to the drawings.

<<First Embodiment>>

A gas composition adjustment apparatus of a first embodiment is provided in a transportation refrigeration apparatus (10). The transportation refrigeration apparatus (10) is provided in a transportation container (1).

The transportation container (1) is a reefer container capable of controlling a temperature in the container. The transportation container (1) is used to transport fresh products (e.g., fruits, vegetables, flowers, and ornamental plants) which breathe by absorbing oxygen ($O_2$) in the air and releasing carbon dioxide ($CO_2$) into the air.

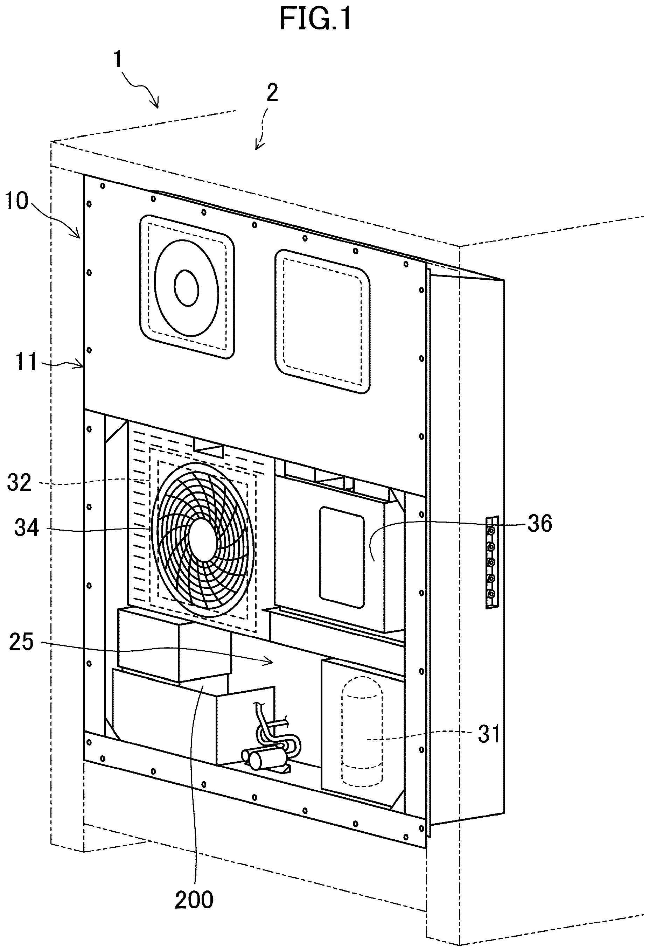

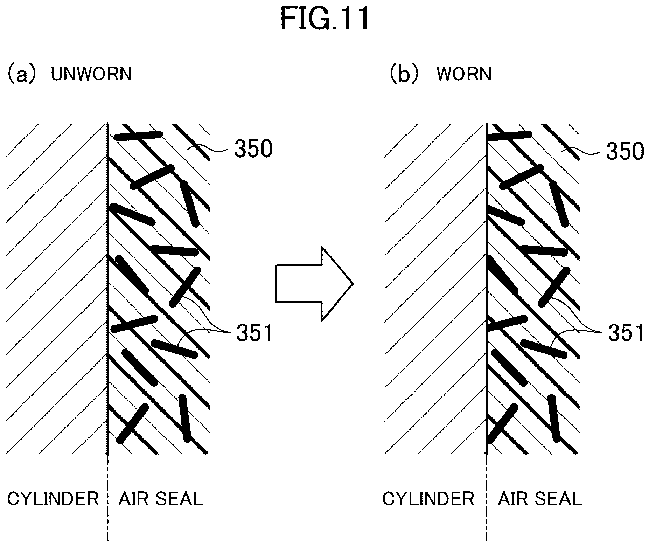

As shown in FIG. 1, the transportation container (1) includes a container body (2) and the transportation refrigeration apparatus (10). The transportation container (1) is used for marine transportation. The transportation container (1) is conveyed by a marine transporter such as a ship.

-Container Body-

The container body (2) is a storage for storing the above fresh products.

The container body (2) has a hollow box shape. The container body (2) is horizontally long. The container body (2) has an opening formed at one end in the longitudinal direction. The opening of the container body (2) is blocked by the transportation refrigeration apparatus (10). The container body (2) contains a storage space (5) for storing products to be transported.

-Transportation Refrigeration Apparatus-

The transportation refrigeration apparatus (10) is attached to the opening of the container body (2). The transportation refrigeration apparatus (10) includes a casing (11) and a refrigerant circuit. The transportation refrigeration apparatus (10) adjusts a temperature of air (inside air) in the storage space (5).

<Casing>

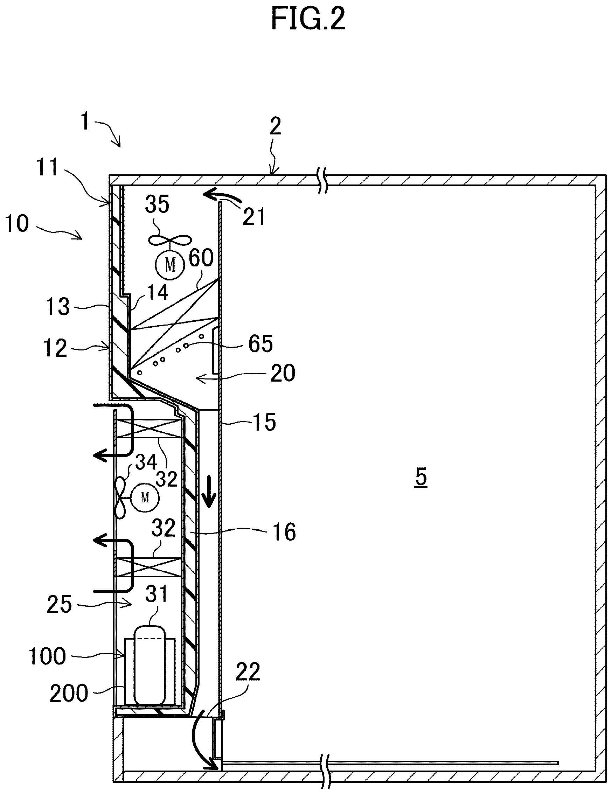

As shown in FIG. 2, the casing (11) includes a division wall (12) and a partition plate (15).

An internal flow path (20) is formed inside the division wall (12). An external chamber (25) is formed outside the division wall (12). The internal flow path (20) and the external chamber (25) are separated by the division wall (12).

The division wall (12) includes an external wall (13) and an internal wall (14). The external wall (13) is located outside the container body (2). The internal wall (14) is located inside the container body (2).

The external wall (13) closes the opening of the container body (2). The external wall (13) is attached to a peripheral portion of the opening of the container body (2). A lower portion of the external wall (13) bulges toward the inside of the container body (2). The external chamber (25) is formed inside the bulging external wall (13).

The internal wall (14) faces the external wall (13). The internal wall (14) is shaped along the external wall (13). The internal wall (14) is spaced apart from the external wall (13). A thermal insulator (16) is provided between the internal wall (14) and the external wall (13).

The partition plate (15) is arranged further inward of the container body (2) than the internal wall (14). The internal flow path (20) is formed between the division wall (12) and the partition plate (15). An inflow port (21) is formed between an upper end of the partition plate (15) and a top panel of the container body (2). An outflow port (22) is formed between a lower end of the partition plate (15) and a lower end of the division wall (12). The internal flow path (20) extends from the inflow port (21) to the outflow port (22).

<Components of Refrigerant Circuit>

The refrigerant circuit is a closed circuit filled with a refrigerant. The refrigerant circuit circulates a refrigerant to perform a vapor compression refrigeration cycle. The refrigerant circuit includes a compressor (31), an external heat exchanger (32), an internal heat exchanger (60), and a refrigerant pipe connecting those components.

The compressor (31) is arranged in a lower portion of the external chamber (25). The external heat exchanger (32) is arranged in an upper portion of the external chamber (25). The external heat exchanger (32) is a fin-and-tube heat exchanger that exchanges heat between a refrigerant and outside air. The external heat exchanger (32) has a generally rectangular tubular shape. The internal heat exchanger (60) is arranged in the internal flow path (20). The internal heat exchanger (60) is a fin-and-tube heat exchanger that exchanges heat between a refrigerant and inside air.

<External Fan>

The transportation refrigeration apparatus (10) includes one external fan (34). The external fan (34) is a propeller fan. The external fan (34) is arranged in the external chamber (25). The external fan (34) is arranged inside the external heat exchanger (32) having a tubular shape. The external fan (34) sends the outside air to the external heat exchanger (32).

<Internal Fan>

The transportation refrigeration apparatus (10) includes two internal fans (35). The internal fans (35) are propeller fans. The internal fans (35) are arranged in the internal flow path (20). The internal fans (35) are arranged above the internal heat exchanger (60). The internal fans (35) supply the inside air to the internal heat exchanger (60).

<Heater>

The transportation refrigeration apparatus (10) includes a heater (65). The heater (65) is arranged below the internal heat exchanger (60). The heater (65) is used to defrost the internal heat exchanger (60).

<Electric Component Box>

As shown in FIG. 1, the transportation refrigeration apparatus (10) includes an electric component box (36). The electric component box (36) is arranged in an upper portion of the external chamber (25). The electric component box (36) houses electric components such as an inverter board and a control board.

-Operation of Transportation Refrigeration Apparatus-

Basic operation of the transportation refrigeration apparatus (10) will be described below. When the transportation refrigeration apparatus (10) operates, the compressor (31), the external fan (34), and the internal fans (35) operate so that the refrigerant circuit performs a refrigeration cycle. In the refrigerant circuit, the external heat exchanger (32) functions as a condenser, and the internal heat exchanger (60) functions as an evaporator. In the external heat exchanger (32), the refrigerant dissipates heat to the outside air to condense. In the internal heat exchanger (60), the refrigerant absorbs heat from the inside air to evaporate. As a result, the internal heat exchanger (60) cools the inside air.

The inside air in the container body (2) circulates between the storage space (5) and the internal flow path (20). The inside air flowing through the internal flow path (20) is cooled by the internal heat exchanger (60). The inside air cooled by the internal heat exchanger (60) is supplied to the storage space (5) through the outflow port (22). In this manner, the inside air in the storage space (5) is cooled, and the temperature of the inside air is kept at a predetermined target temperature.

-Gas Composition Adjustment Apparatus-

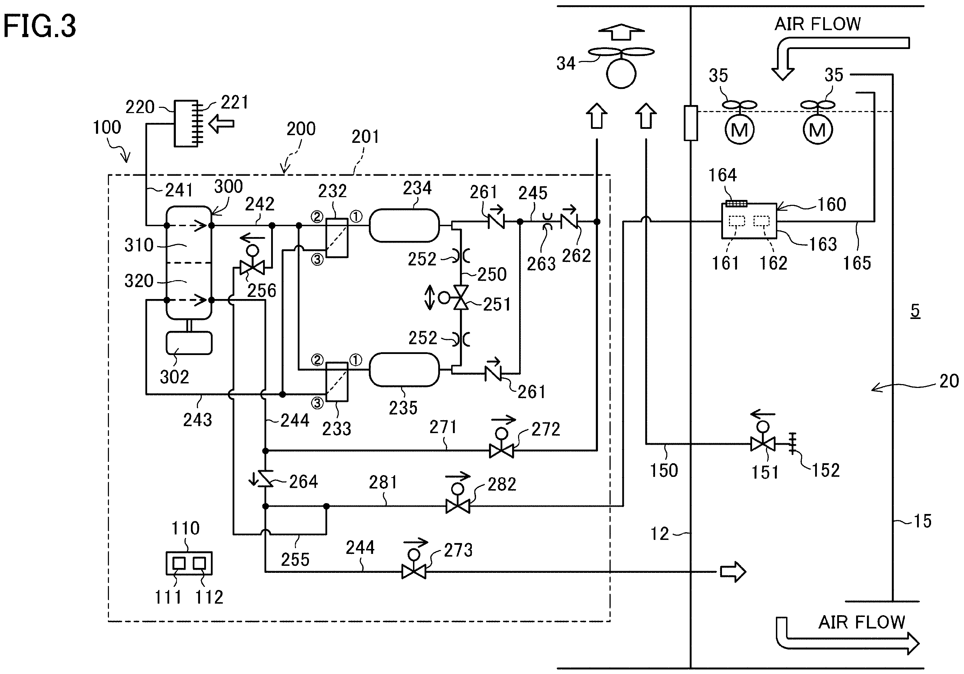

The gas composition adjustment apparatus (100) of this embodiment will be described.
The gas composition adjustment apparatus (100) is provided in the transportation refrigeration apparatus (10) to perform what is called "controlled atmosphere (CA) transport." The gas composition adjustment apparatus (100) adjusts a composition (specifically, concentrations of nitrogen, oxygen, and carbon dioxide) of the air in the storage space (5) of the transportation container (1) to a composition different from a composition of the atmospheric air.
As shown in FIG. 3, the gas composition adjustment apparatus (100) includes a filter unit (220), a main unit (200), a sensor unit (160), a ventilation exhaust pipe (150), and a controller (110). The gas composition adjustment apparatus (100) separates outside air (atmospheric air), which is gas to be treated, into nitrogen-enriched gas and oxygen-enriched gas by what are called "pressure swing adsorption (PSA)," where the nitrogen-enriched gas has a higher nitrogen concentration and a lower oxygen concentration than the atmospheric air, and the oxygen-enriched gas has a lower nitrogen concentration and a higher oxygen concentration than the atmospheric air. The nitrogen-enriched gas serves as a second gas. The oxygen-enriched gas serves as a first gas.

<Filter Unit and Outside Air Pipe>

The filter unit (220) is a member having a box shape. The filter unit (220) is installed in the external chamber (25) of the transportation refrigeration apparatus (10). The filter unit (220) includes an air filter (221). The air filter (221) is a filter for capturing dust, salt, and the like contained in the outside air. The air filter (221) of this embodiment is an air-permeable, waterproof membrane filter.
The filter unit (220) is connected to the main unit (200) via an outside air pipe (241). One end of the outside air pipe (241) is connected to the filter unit (220). The other end of the outside air pipe (241) is connected to an air pump (300) which will be described later. The outside air pipe (241) guides the outside air (atmospheric air) having passed through the air filter (221), as a gas to be treated, to the air pump (300).

<Main Unit>

The main unit (200) is installed in the external chamber (25) of the transportation refrigeration apparatus (10). The main unit (200) includes the air pump (300), a first adsorption vessel (234), a second adsorption vessel (235), a first switching valve (232), a second switching valve (233), and a unit case (201) housing those components. The unit case (201) is provided with an introduction pipe (242), a suction pipe (243), a first outflow pipe (244), and a second outflow pipe (245).

<Air Pump>

The air pump (300) includes a supply pump (310), a discharge pump (320), and a drive motor (302). The supply pump (310) and the discharge pump (320) each suck and discharge air. The supply pump (310) and the discharge pump (320) are connected to a drive shaft of the single drive motor (302). In the air pump (300), both the supply pump (310) and the discharge pump (320) are driven by the single drive motor (302).

The supply pump (310) includes a suction port connected to the other end of the outside air pipe (241). The supply pump (310) includes a discharge port connected to one end of the introduction pipe (242). The supply pump (310) supplies gas to be treated, which has been sucked from the outside air pipe (241), to the first adsorption vessel (234) and the second adsorption vessel (235) through the introduction pipe (242).

The discharge pump (320) includes a suction port connected to the suction pipe (243). The discharge pump (320) includes a discharge port connected to the first outflow pipe (244). The discharge pump (320) supplies gas, which has been sucked from the first adsorption vessel (234) and the second adsorption vessel (235) through the suction pipe (243), to the storage space (5) of the container body (2) through the first outflow pipe (244).

introduction Pipe>

The introduction pipe (242) is a pipe for guiding gas to be treated, which has been discharged from the supply pump (310), to the first adsorption vessel (234) and the second adsorption vessel (235). One end of the introduction pipe (242) is connected to the discharge port of the supply pump (310). The other end of the introduction pipe (242) branches into two branch pipes, one of which is connected to the first switching valve (232) and the other one of which is connected to the second switching valve (233).

<Suction Pipe>

The suction pipe (243) is a pipe for guiding gas, which has flown out from the first adsorption vessel (234) and the second adsorption vessel (235), to the discharge pump (320). One end of the suction pipe (243) is connected to the suction port of the discharge pump (320). The other end of the suction pipe (243) branches into two branch pipes, one of which is connected to the first switching valve (232) and the other one of which is connected to the second switching valve (233).

<First Outflow Pipe>

The first outflow pipe (244) is a pipe for guiding gas, which has been discharged from the discharge pump (320), to the internal flow path (20). One end of the first outflow pipe (244) is connected to the discharge port of the discharge pump (320). The other end of the first outflow pipe (244) opens to a portion downstream of the internal fans (35) in the internal flow path (20).

The first outflow pipe (244) includes a check valve (264) and a supply-side on-off valve (273) in this order between end to end. The check valve (264) allows air to flow only from one end to the other end of the first outflow pipe (244) and blocks air from flowing in the reverse direction. The supply-side on-off valve (273) is an on-off valve comprised of an electromagnetic valve.

<Switching Valve>

The first switching valve (232) and the second switching valve (233) are switching valves each having three ports. The first switching valve (232) and the second switching valve (233) are each configured to switch between a first state where a first port communicates with a second port so as to be blocked from a third port, and a second state where the first port communicates with the third port so as to be blocked from the second port.

The first switching valve (232) has the first port connected to one end of the first adsorption vessel (234). The first switching valve (232) has the second port connected to one of the branch pipes of the introduction pipe (242), and the third port connected to one of the branch pipes of the suction pipe (243). The first switching valve (232) switches between a state where the first adsorption vessel (234) is connected to the supply pump (310) and a state where the first adsorption vessel (234) is connected to the discharge pump (320).

The second switching valve (233) has the first port connected to one end of the second adsorption vessel (235). The second switching valve (233) has the second port connected to one of the branch pipes of the introduction pipe (242), and the third port connected to one of the branch pipes of the suction pipe (243). The second switching valve (233) switches between a state where the second adsorption vessel (235) is connected to the supply pump (310) and a state where the second adsorption vessel (235) is connected to the discharge pump (320).

<Adsorption Vessel>

The first adsorption vessel (234) and the second adsorption vessel (235) are members each including a cylindrical container and an adsorbent, where both ends of the cylindrical container are closed and the adsorbent fills the container. Each adsorption vessel (234, 235) is an adsorber containing an adsorbent.

The adsorbent that fills each adsorption vessel (234, 235) has characteristics of adsorbing nitrogen and water (water vapor) in gas to be treated in a pressurized state with a pressure higher than the atmospheric pressure, and desorbing nitrogen and water in a depressurized state with a pressure lower than the atmospheric pressure. One example of an adsorbent having such characteristics is porous zeolite with pores having a diameter smaller than the diameter of nitrogen molecules (3.0 angstrom) and larger than the diameter of oxygen molecules (2.8 angstrom). The adsorbent in each adsorption vessel (234, 235) adsorbs nitrogen and water (water vapor) which are the components of gas to be treated.

<Second Outflow Pipe>

One end of the second outflow pipe (245) branches into two branch pipes, one of which is connected to the other end of the first adsorption vessel (234) and the other of which is connected to the other end of the second adsorption vessel (235). Each branch pipe of the second outflow pipe (245) is provided with one check valve (261). Each check valve (261) allows air to flow in a direction in which the air flows out from the associated adsorption vessel (234, 235) and blocks air from flowing in the reverse direction.

The second outflow pipe (245) extends to the outside of the unit case (201). The other end of the second outflow pipe (245) opens to the external chamber (25) of the transportation container (1). The second outflow pipe (245) has a joined portion provided with a check valve (262) and an orifice (263). The check valve (262) is disposed closer to the other end of the second outflow pipe (245) than the orifice (263) is. This check valve (262) allows air to flow toward the other end of the second outflow pipe (245) and blocks air from flowing in the reverse direction.

<Purge Pipe>

The branch pipes of the second outflow pipe (245) are connected to a purge pipe (250). The purge pipe (250) has ends, one of which is connected to the branch pipe connected to the first adsorption vessel (234), and the other one of which is connected to the branch pipe connected to the second adsorption vessel (235). The one end of the purge pipe (250) is connected between the first adsorption vessel (234) and the associated check valve (261). The other end of the purge pipe (250) is connected between the second adsorption vessel (235) and the associated check valve (261).

The purge pipe (250) is provided with a purge valve (251). The purge valve (251) is an on-off valve comprised of an electromagnetic valve. The purge valve (251) is opened to equalize pressures of the first adsorption vessel (234) and the second adsorption vessel (235). An orifice (252) is provided on each side of the purge valve (251) of the purge pipe (250).

<Exhaust Connection Pipe>

The first outflow pipe (244) is connected with an exhaust connection pipe (271). The exhaust connection pipe (271) has ends, one of which is connected to the first outflow pipe (244) and the other one of which is connected to the second outflow pipe (245). The one end of the exhaust connection pipe (271) is connected to the first outflow pipe (244) between the discharge pump (320) and the check valve (264). The other end of the exhaust connection pipe (271) is connected to the second outflow pipe (245) that is closer to the outside of the container than the check valve (262) is.

The exhaust connection pipe (271) is provided with an exhaust on-off valve (272). The exhaust on-off valve (272) is an on-off valve comprised of an electromagnetic valve. The exhaust on-off valve (272) is opened to discharge air flowing through the first outflow pipe (244) to the outside of the container.

<Measurement Pipe>

The first outflow pipe (244) is connected with a measurement pipe (281). The measurement pipe (281) is a pipe for connecting the first outflow pipe (244) to the sensor unit (160). The measurement pipe (281) has ends, one of which is connected to the first outflow pipe (244) between the check valve (264) and the supply-side on-off valve (273). The other end of the measurement pipe (281) is connected to the sensor unit (160).

The measurement pipe (281) is provided with a measurement on-off valve (282). The measurement on-off valve (282) is an on-off valve comprised of an electromagnetic valve. The measurement on-off valve (282) is opened to send the air flowing through the first outflow pipe (244) to the sensor unit (160).

<Bypass Pipe>

The introduction pipe (242) is connected with a bypass connection pipe (255). The bypass connection pipe (255) is a pipe for enabling outside air to bypass the first adsorption vessel (234) and the second adsorption vessel (235) and to be supplied to the storage space (5) of the transportation container (1). The bypass connection pipe (255) has ends, one of which is connected between the branch point of the introduction pipe (242) and the supply pump (310). The other end of the bypass connection pipe (255) is connected between the one end of the measurement pipe (281) and the measurement on-off valve (282).

The bypass connection pipe (255) is provided with a bypass on-off valve (256). The bypass on-off valve (256) is an on-off valve comprised of an electromagnetic valve. This bypass on-off valve (256) is opened to supply outside air discharged by the supply pump (310) to the storage space (5) without changing the composition of the outside air.

<Sensor Unit>

The sensor unit (160) includes an oxygen sensor (161), a carbon dioxide sensor (162), and a sensor case (163).

The oxygen sensor (161) is, e.g., a sensor of a zirconia current type that measures an oxygen concentration in mixed gas such as air. The carbon dioxide sensor (162) is, e.g., a non-dispersive infrared (NDIR) sensor that measures a carbon dioxide concentration in mixed gas such as air. The oxygen sensor (161) and the carbon dioxide sensor (162) are housed in the sensor case (163).

The sensor case (163) is a box-shaped member. The sensor case (163) includes an air filter (164). The air filter (164) is a membrane filter for capturing dust and the like contained in the inside air. The air filter (164) filters the inside air flowing into the sensor case (163).

The sensor case (163) is connected with the measurement pipe (281). The sensor case (163) is connected with an outlet pipe (165). The outlet pipe (165) has an inlet end connected to the sensor case (163), and an outlet end opened upstream of the internal fans (35) in the internal flow path (20).

When the measurement on-off valve (282) is closed, the inside air flows inside the sensor case (163). Specifically, the inside air flowing in the internal flow path (20) flows into the sensor case (163) through the air filter (164). After having passed through the sensor case (163), the inside air flows through the outlet pipe (165), and then flows into the suction side of the internal fans (35) in the internal flow path (20). Thus, when the measurement on-off valve (282) is closed, the oxygen sensor (161) measures an oxygen concentration in the inside air, and the carbon dioxide sensor (162) measures a carbon dioxide concentration in the inside air.

On the other hand, when the measurement on-off valve (282) is open, the gas flowing in the measurement pipe (281) flows inside the sensor case (163). Specifically, the gas flowing in the first outflow pipe (244) or the bypass connection pipe (255) flows into the sensor case (163) through the measurement pipe (281). After having passed through the sensor case (163), the gas flows through the outlet pipe (165), and then flows into the suction-side of the internal fans (35) in the internal flow path (20). Thus, when the measurement on-off valve (282) is open, the oxygen sensor (161) measures an oxygen concentration in the gas flowing from the measurement pipe (281) into the sensor case (163), and the carbon dioxide sensor (162) measures a carbon dioxide concentration in the gas flowing from the measurement pipe (281) into the sensor case (163).

<Ventilation Exhaust Pipe>

The ventilation exhaust pipe (150) is a pipe for discharging inside air in the transportation container (1) to the external space. The ventilation exhaust pipe (150) penetrates the division wall (12) of the transportation refrigeration apparatus (10). The ventilation exhaust pipe (150) is provided with a ventilation exhaust valve (151). The ventilation exhaust valve (151) is an on-off valve comprised of an electromagnetic valve.

<Controller>

The controller (110) includes a microcomputer (111) mounted on a control board, and a memory device (112) storing software for operating the microcomputer (111). The memory device (112) is a semiconductor memory.

The controller (110) controls the components of the gas composition adjustment apparatus (100). The controller (110) receives values measured by the oxygen sensor (161) and the carbon dioxide sensor (162). The controller (110) controls the air pump (300), a first switching valve (136), and a second switching valve (137). The controller (110) controls the ventilation exhaust valve (151), the purge valve (251), the bypass on-off valve (256), the exhaust on-off valve (272), the supply-side on-off valve (273), and the measurement on-off valve (282).

-Operation of Gas Composition Adjustment Apparatus-

The gas composition adjustment apparatus (100) adjusts the composition of the inside air (in this embodiment, an oxygen concentration and a carbon dioxide concentration in the inside air) in the storage space (5) of the transportation container (1). Here, an operation of the gas composition adjustment apparatus (100) will be described with reference to an example where a target range of the oxygen concentration in the inside air is 5% ± 1% and a target range of the carbon dioxide concentration in the inside air is 2% ± 1%.

The gas composition adjustment apparatus (100) of this embodiment performs an adjustment operation to reduce an oxygen concentration and a carbon dioxide concentration in the inside air in the storage space (5), and performs an outside air introduction operation to increase an oxygen concentration in the inside air in the storage space (5). In the adjustment operation, the gas composition adjustment apparatus (100) supplies, to the storage space (5), the nitrogen-enriched gas produced from the outside air (atmospheric air) which is gas to be treated. In the outside air introduction operation, the gas composition adjustment apparatus (100) supplies untreated outside air (atmospheric air) to the storage space (5).

The controller (110) of the gas composition adjustment apparatus (100) decides to start and stop the adjustment operation and decides to start and stop the outside air introduction operation based on the values measured by the oxygen sensor (161) and the carbon dioxide sensor (162).

The composition of air in the storage space (5) at the time when loading the transportation container (1) with cargos (6) is completed is substantially the same as the composition of the atmospheric air (a nitrogen concentration: 78%, an oxygen concentration: 21%, and a carbon dioxide concentration: 0.04%). Thus, the gas composition adjustment apparatus (100) performs the adjustment operation to reduce an oxygen concentration in the inside air. When the oxygen concentration in the inside air reaches the upper limit (6%) of the target range, the gas composition adjustment apparatus (100) stops the adjustment operation.

After the oxygen concentration in the inside air has reached 6% and the adjustment operation of the gas composition adjustment apparatus (100) has stopped, the oxygen concentration in the inside air gradually decreases and the carbon dioxide concentration in the inside air gradually increases due to respiration of the fresh products contained in the storage space (5).

When the carbon dioxide concentration in the inside air reaches the upper limit (3%) of the target range, the gas composition adjustment apparatus (100) performs the adjustment operation to reduce a carbon dioxide concentration in the inside air. When the carbon dioxide concentration in the inside air reaches the lower limit (1%) of the target range, the gas composition adjustment apparatus (100) stops the adjustment operation.

When the oxygen concentration in the inside air reaches the lower limit (4%) of the target range, the gas composition adjustment apparatus (100) performs the outside air introduction operation to increase an oxygen concentration in the inside air. When the oxygen concentration in the inside air reaches the upper limit (6%) of the target range, the gas composition adjustment apparatus (100) stops the outside air introduction operation.

In this manner, the gas composition adjustment apparatus (100) performs the adjustment operation to reduce an oxygen concentration in the inside air in the storage space (5) from 21% (the oxygen concentration in the atmospheric air) to the target range. The gas composition adjustment apparatus (100) repeatedly performs the adjustment operation and the outside air introduction operation to keep the oxygen concentration and the carbon dioxide concentration in the inside air in the storage space (5) within the respective target ranges.

-Adjustment Operation of Gas Composition Adjustment Apparatus-

In the adjustment operation, the gas composition adjustment apparatus (100) separates the outside air (atmospheric air) as a gas to be treated into nitrogen-enriched gas and oxygen-enriched gas, supplies the nitrogen-enriched gas to the storage space (5), and discharges the oxygen-enriched gas to the external space. In the adjustment operation, the ventilation exhaust valve (151) is opened, and the inside air is discharged to the external space through the ventilation exhaust pipe (150).

In the adjustment operation, a flow rate of the nitrogen-enriched gas supplied to the storage space (5) by the gas composition adjustment apparatus (100) is higher than that of the inside air discharged from the storage space (5) by the gas composition adjustment apparatus (100). Thus, the storage space (5) is kept at a positive pressure.

In the adjustment operation, the gas composition adjustment apparatus (100) performs the first operation and the second operation alternately for a predetermined switching time period Ts each. The switching time Ts is set to, e.g., 14 seconds. The controller (110) controls the first switching valve (232) and the second switching valve (233) so that the first operation and the second operation are performed alternately.

<First Operation>

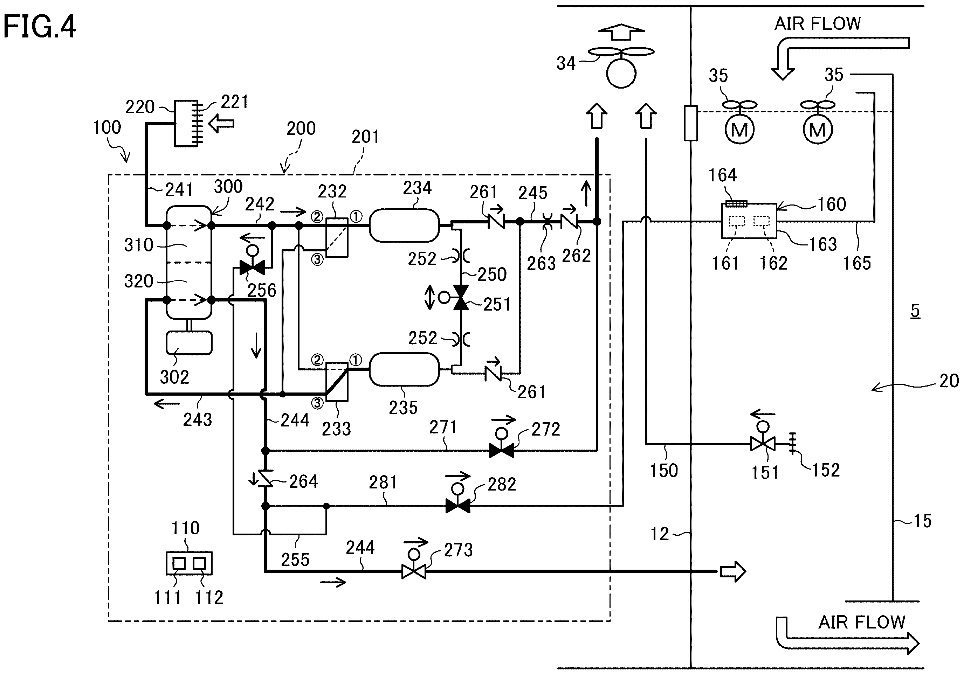

As shown in FIG. 4, in the first operation, the first switching valve (232) is set to the first state, and the second switching valve (233) is set to the second state. In the first operation, the supply-side on-off valve (273) is opened, and the remaining on-off valves (251, 256, 272, 282) are closed. In the first operation, the air pump (300) operates to perform a pressurization operation for the first adsorption vessel (234) and a depressurization operation for the second adsorption vessel (235).

The supply pump (310) sucks gas to be treated from the outside air pipe (241), pressurizes the sucked gas, and supplies the pressurized gas to the first adsorption vessel (234). In the first adsorption vessel (234), the nitrogen and water (water vapor) contained in the supplied gas are adsorbed by the adsorbent. As a result, in the first adsorption vessel (234), an oxygen-enriched gas having a lower nitrogen concentration and a higher oxygen concentration than those of the gas to be treated is produced. The oxygen-enriched gas flows out from the first adsorption vessel (234), flows through the second outflow pipe (245), and is discharged to the external space.

On the other hand, the discharge pump (320) sucks gas from the second adsorption vessel (235). In the second adsorption vessel (235), the internal pressure decreases and the nitrogen and water are desorbed from the adsorbent. As a result, in the second adsorption vessel (235), a nitrogen-enriched gas having a higher nitrogen concentration and a lower oxygen concentration than those of the gas to be treated is produced. The nitrogen-enriched gas flows from the second adsorption vessel (235) into the suction pipe (243) and is sucked into the discharge pump (320). The discharge pump (320) pressurizes the sucked nitrogen-enriched gas and discharges the pressurized nitrogen-enriched gas to the first outflow pipe (244). The nitrogen-enriched gas flows through the first outflow pipe (244) and is supplied to the internal flow path (20).

<Second Operation>

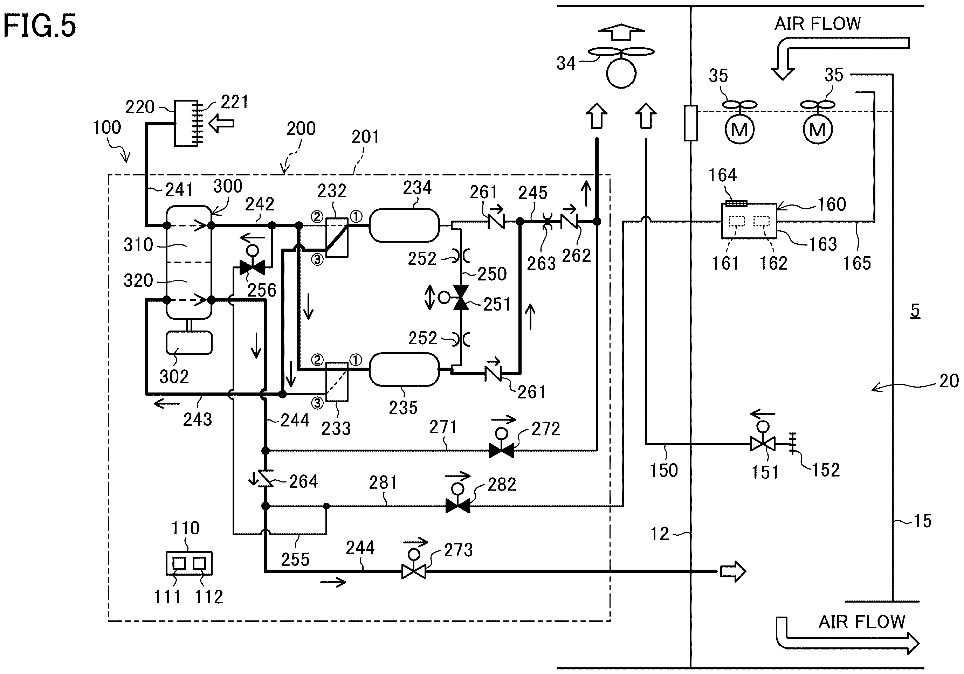

As shown in FIG. 5, in the second operation, the first switching valve (232) is set to the second state, and the second switching valve (233) is set to the first state. In the second operation, the supply-side on-off valve (273) is opened, and the remaining on-off valves (251, 256, 272, 282) are closed. In the second operation, the air pump (300) operates to perform a depressurization operation for the first adsorption vessel (234) and a pressurization operation for the second adsorption vessel (235).

The supply pump (310) sucks gas to be treated from the outside air pipe (241), pressurizes the sucked gas, and supplies the pressurized gas to the second adsorption vessel (235). In the second adsorption vessel (235), the nitrogen and water (water vapor) contained in the supplied gas are adsorbed by the adsorbent. As a result, in the second adsorption vessel (235), an oxygen-enriched gas having a lower nitrogen concentration and a higher oxygen concentration than those of the gas to be treated is produced. The oxygen-enriched gas flows out from the second adsorption vessel (235), flows through the second outflow pipe (245), and is discharged to the external space.

On the other hand, the discharge pump (320) sucks gas from the first adsorption vessel (234). In the first adsorption vessel (234), the internal pressure decreased, and the nitrogen and water are desorbed from the adsorbent. As a result, in the first adsorption vessel (234), a nitrogen-enriched gas with a higher nitrogen concentration and a lower oxygen concentration than those of the gas to be treated is produced. The nitrogen-enriched gas flows from the first adsorption vessel (234) into the suction pipe (243) and is sucked into the discharge pump (320). The discharge pump (320) pressurizes the sucked nitrogen-enriched gas and discharges the pressurized nitrogen-enriched gas to the first outflow pipe (244). The nitrogen-enriched gas flows through the first outflow pipe (244) and is supplied to the internal flow path (20).

-Air Pump-

The air pump (300) will be described in detail. As described above, the air pump (300) includes the supply pump (310) and the discharge pump (320). The supply pump (310) and the discharge pump (320) are positive displacement pumps. The supply pump (310) and the discharge pump (320) are oilless pumps that do not use any lubricant.

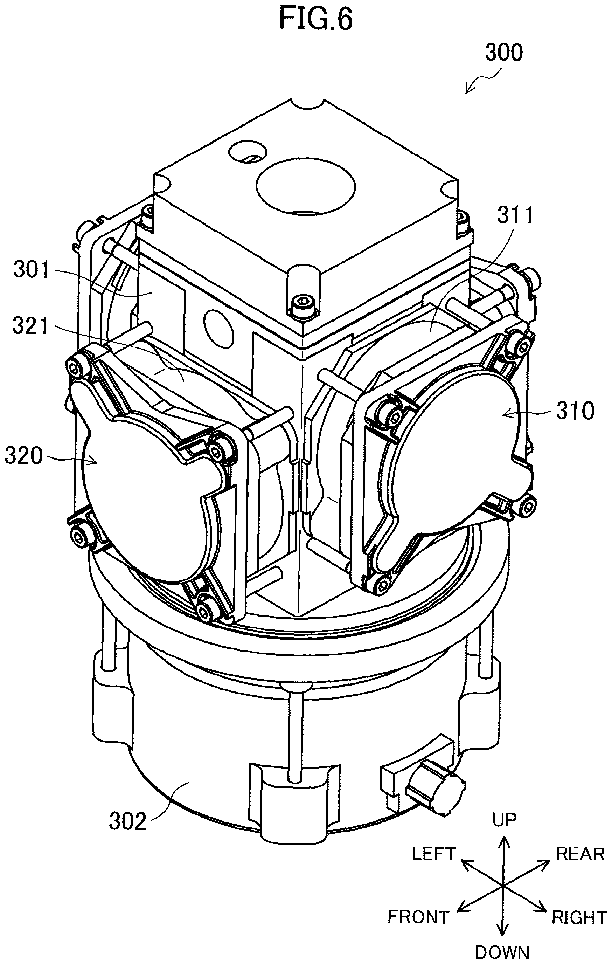

As shown in FIGS. 6 to 8, the air pump (300) includes one crankcase (301) and one drive motor (302). The air pump (300) includes two supply-side cylinders (311), two supply-side pistons (312), two discharge-side cylinders (321), and two discharge-side pistons (322). Note that the terms "up", "down", "right", "left", "front", and "rear" used in the description of the air pump (300) refer to the directions shown in FIG. 6 (specifically, the directions when the air pump (300) is viewed from the front).

The crankcase (301) is a member having a quadrangular prism shape. Although not shown, the crankcase (301) houses a crankshaft. The drive motor (302) is attached to the lower end of the crankcase (301). The drive motor (302) is connected to the crankshaft and drives the crankshaft.

<Supply Pump>

In the air pump (300), the two supply-side cylinders (311) and the two supply-side pistons (312) constitute the supply pump (310). One of the supply-side cylinders (311) is paired with one of the supply-side pistons (312). The other one of the supply-side cylinders (311) is paired with the other one of the supply-side pistons (312).

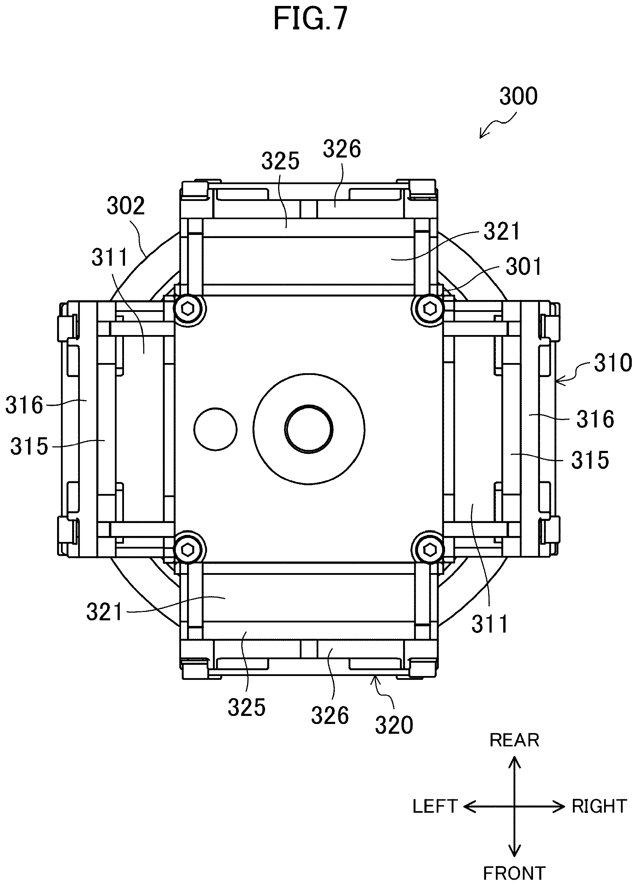

The supply-side cylinders (311) are short cylindrical members. As shown in FIG. 7, the supply-side cylinder (311) is attached to each of the right and left side surfaces of the crankcase (301). The proximal end of the supply-side cylinder (311) is in close contact with the side surface of the crankcase (301).

To each supply-side cylinder (311), a cylinder head (315) and a head cover (316) are attached. The cylinder head (315) is a rectangular plate-shaped member. The cylinder head (315) closes the distal end of the supply-side cylinder (311). The cylinder head (315) includes a suction port (315a) and a discharge port (315b). The head cover (316) is a rectangular plate-shaped member. The head cover (316) covers the associated cylinder head (315).

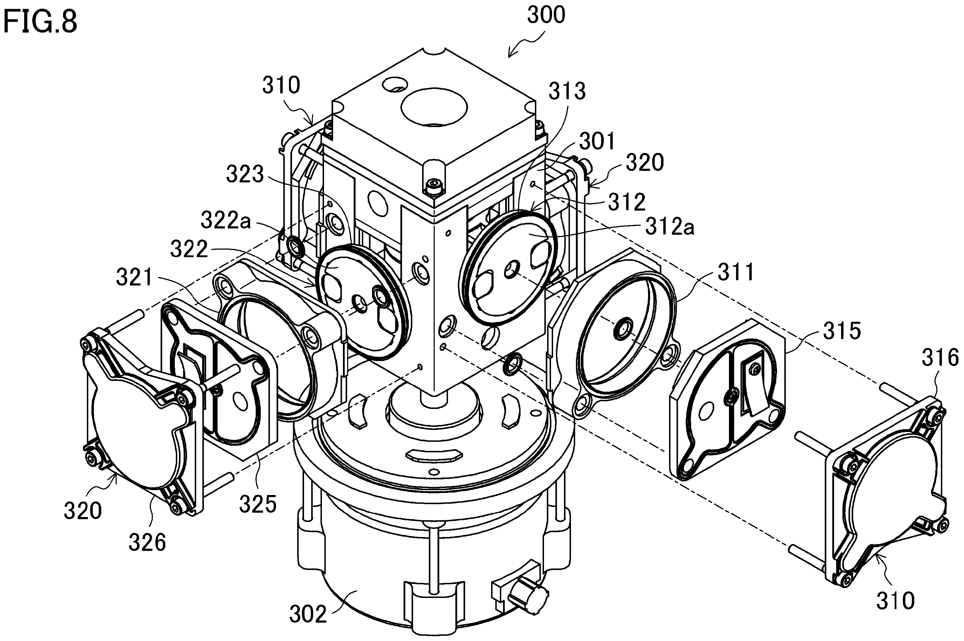

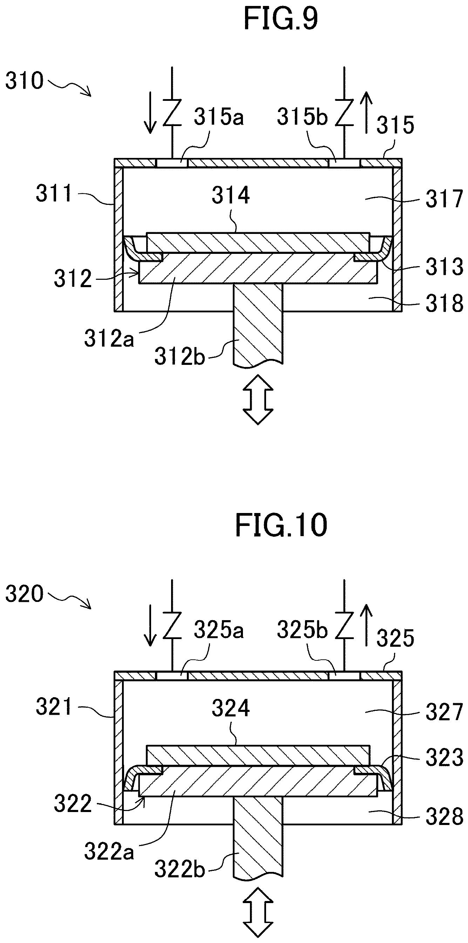

As shown in FIGS. 8 and 9, each supply-side piston (312) is accommodated in the associated supply-side cylinder (311). Each supply-side piston (312) is connected to the crankshaft. When the crankshaft rotates, each supply-side piston (312) reciprocates along the axis of the associated supply-side cylinder (311).

As shown in FIG. 9, the supply-side piston (312) includes a piston body (312a), a rod (312b), a supply-side air seal (313), and a seal fixing plate (314).

The piston body (312a) is a disk-shaped metal member. The rod (312b) is a rod-shaped metal member. The rod (312b) is integrated with the piston body (312a).

The rod (312b) is connected to the crankshaft.

The supply-side air seal (313) is a flexible, ring-shaped member. The supply-side air seal (313) has an outer circumferential edge that curves throughout the entire circumference toward the cylinder head (315). The supply-side air seal (313) is a sealing member. The supply-side air seal (313) seals a gap between the piston body (312a) and the inner wall surface of the supply-side cylinder (311) in order to keep the hermeticity of a gas chamber (317).

The seal fixing plate (314) is a disk-shaped metal member. The seal fixing plate (314) is fixed to the piston body (312a) by bolts or the like, and holds the supply-side air seal (313) between the seal fixing plate (314) and the piston body (312a).

In the supply pump (310), the internal space of the supply-side cylinder (311) is divided into the gas chamber (317) closer to the cylinder head (315) and a back-pressure chamber (318) closer to the crankcase (301) by the piston body (312a) of the supply-side piston (312). When the supply-side piston (312) reciprocates, gas is sucked from the suction port (315a) into the gas chamber (317), and the gas in the gas chamber (317) is discharged from the discharge port (315b).

<Discharge Pump>

In the air pump (300), the two discharge-side cylinders (321) and the two discharge-side pistons (322) constitute the discharge pump (320). One of the discharge-side cylinders (321) is paired with one of the discharge-side pistons (322). The other one of the discharge-side cylinders (321) is paired with the other one of the discharge-side pistons (322).

The discharge-side cylinders (321) are short cylindrical members. As shown in FIG. 7, the discharge-side cylinder (321) is attached to each of the front and rear side surfaces of the crankcase (301). The proximal end of the discharge-side cylinder (321) is in close contact with the side surface of the crankcase (301).

To each discharge-side cylinder (321), a cylinder head (325) and a head cover (326) are attached. The cylinder head (325) is a rectangular plate-shaped member. The cylinder head (325) closes the distal end of the associated discharge-side cylinder (321). The cylinder head (325) includes a suction port (325a) and a discharge port (325b). The head cover (326) is a rectangular plate-shaped member. The head cover (326) covers the cylinder head (325).

As shown in FIGS. 8 and 10, each discharge-side piston (322) is accommodated in the associated discharge-side cylinder (321). Each discharge-side piston (322) is connected to the crankshaft. When the crankshaft rotates, each discharge-side piston (322) reciprocates along the axis of the associated discharge-side cylinder (321).

As shown in FIG. 10, the discharge-side piston (322) includes a piston body (322a), a rod (322b), a discharge-side air seal (323), and a seal fixing plate (324).

The piston body (322a) is a disk-shaped metal member. The rod (322b) is a rod-shaped metal member. The rod (322b) is integrated with the piston body (322a). The rod (322b) is connected to the crankshaft.

The discharge-side air seal (323) is a flexible, ring-shaped member. The discharge-side air seal (323) has an outer circumferential edge that curves throughout the entire circumference toward the discharge-side piston (322). The discharge-side air seal (323) is a sealing member. The discharge-side air seal (323) seals a gap between the piston body (322a) and the inner wall surface of the discharge-side cylinder (321) to keep the hermeticity of a gas chamber (327).

The seal fixing plate (324) is a disk-shaped metal member. The seal fixing plate (324) is fixed to the piston body (322a) by bolts or the like, and holds the discharge-side air seal (323) between the seal fixing plate (324) and the piston body (322a).

In the discharge pump (320), the internal space of each discharge-side cylinder (321) is divided into the gas chamber (327) closer to the cylinder head (325) and a back-pressure chamber (328) closer to the crankcase (301) by the piston body (322a) of the discharge-side piston (322). When the discharge-side piston (322) reciprocates, gas is sucked from the suction port (325a) into the gas chamber (327), and the gas in the gas chamber (327) is discharged through the discharge port (325b).

<Material and Characteristics of Air Seal>

The main components of the supply-side air seal (313) are a fluororesin and a particulate carbon material. The fluororesin of the supply-side air seal (313) is polytetrafluoroethylene (PTFE). The particulate carbon material of the supply-side air seal (313) is particulate carbon with an average particle size of about 35 nm. The supply-side air seal (313) contains 75% by mass of PTFE and 23% by mass of the particulate carbon material. The supply-side air seal (313) has a linear expansion coefficient expressed by $7.7 \times 10^{-5}$ [1/°C] along the radius. The supply-side air seal (313) has a Young's modulus of 0.67 [GPa] at a temperature of 75°C.

The main components of the discharge-side air seal (323) are a fluororesin and a fibrous carbon material. The fluororesin of the discharge-side air seal (323) is polytetrafluoroethylene (PTFE). The fibrous carbon material of the discharge-side air seal (323) has a diameter of about 5 $\mu$m to about 15 $\mu$m and a length of about 20 $\mu$m to about 30 $\mu$m. The discharge-side air seal (323) contains 90% by mass of PTFE and 10% by mass of the particulate carbon material. The discharge-side air seal (323) has a linear expansion coefficient expressed by $9.3 \times 10^{-5}$ [1/°C] along the radius. The discharge-side air seal (323) has a Young's modulus of 0.52 [GPa] at a temperature of 75°C.

-Wear of Air Seal-

<Supply-Side Air Seal>

As shown in FIG. 9, in the supply pump (310), the outer circumferential edge of the supply-side air seal (313) slides on the inner wall surface of the supply-side cylinder (311).

When the supply pump (310) operates, the pressure of the gas chamber (317) becomes higher than the pressure of the back-pressure chamber (318). Thus, the outer circumferential edge of the supply-side air seal (313) is pressed and expanded by the gas pressure of the gas chamber (317), and is pressed onto the inner wall surface of the supply-side cylinder (311). When the supply pump (310) operates, the supply-side cylinder (311) has a temperature of about 80°C, and the supply-side air seal (313) also has a temperature close to 80°C. Thus, when the supply pump (310) operates, the supply-side air seal (313) thermally expands, such that the outer circumferential edge of the supply-side air seal (313) is pressed onto the inner wall surface of the supply-side cylinder (311).

While being pressed onto the inner wall surface of the supply-side cylinder (311), the supply-side air seal (313) reciprocates together with the associated supply-side piston (312). Thus, the outer circumferential edge of the supply-side air seal (313) gradually wears.

<Dischage-Side Air Seal>

As shown in FIG. 10, in the discharge pump (320), the outer circumferential edge of the discharge-side air seal (323) slides on the inner wall surface of the discharge-side cylinder (321).

When the discharge pump (320) operates, the pressure of the back-pressure chamber (328) becomes higher than the pressure of the gas chamber (327). Thus, the outer circumferential edge of the discharge-side air seal (323) is pressed and expanded by the gas pressure of the back-pressure chamber (328), and is pressed onto the inner wall surface of the discharge-side cylinder (321).

When the discharge pump (320) operates, the discharge-side cylinder (321) has a temperature of about 65°C, and the discharge-side air seal (323) also has a temperature close to 65°C. Thus, when the discharge pump (320) operates, the discharge-side air seal (323) thermally expands, such that the outer circumferential edge of the discharge-side air seal (323) is pressed onto the inner wall surface of the discharge-side cylinder (321).

While being pressed onto the inner wall surface of the discharge-side cylinder (321), the discharge-side air seal (323) reciprocates together with the associated discharge-side piston (322). Thus, the outer circumferential edge of the discharge-side air seal (323) gradually wears.

-Selection of Dicharge-Side Air Seal-

As shown in FIG. 11, an air seal contains a fluororesin (350) and a fibrous carbon material (351) as main components, where the fibrous carbon material (351) is dispersed in the fluororesin (350). As the air seal wears, the fibrous carbon material (351) comes into contact with the wall surface of the cylinder and is abraded by the cylinder.

The fibrous carbon material (351) has an elongated shape. Thus, even if the fibrous carbon material (351) is partially exposed on the surface of the air seal and comes into contact with the cylinder, the remaining part thereof continues to be is held by the fluororesin (350). In the air seal containing the fibrous carbon material (351), the fibrous carbon material (351) continuously exhibits the wear reduction effect.

On the other hand, when the air seal containing a fluororesin and a particulate carbon material as main components wears, and the particulate carbon materials come into contact with the cylinder, the particulate carbon material falls off from the air seal. When falling off from the air seal, the particulate carbon material exhibits less wear reduction effect, and the air seal is more likely to wear.

Thus, if the air seal used as the discharge-side air seal (323) is switched from an "air seal containing a fluororesin and a particulate carbon material as main components" to an "air seal containing a fluororesin and a fibrous carbon material as main components," the discharge-side air seal (323) has a longer life thanks to the wear reduction effect of a fibrous carbon material.

-Selection of Supply-Side Air Seal-

In consideration of the "wear reduction effect of a fibrous carbon material" described above, if the air seal used as the supply-side air seal (313) is switched from an "air seal containing a fluororesin and a particulate carbon material as main components" to an "air seal containing a fluororesin and a fibrous carbon material as main components," the supply-side air seal (313) is expected to have a longer life.

However, if an "air seal containing a fluororesin and a fibrous carbon material as main components" is used as the supply-side air seal (313), the supply-side air seal (313) has a shorter life than if an "air seal containing a fluororesin and a particulate carbon material as main components" is used as the supply-side air seal (313). Thus, the air pump of this embodiment employs an "air seal containing a fluororesin and a particulate carbon material as main components" as the supply-side air seal (313). The reasons will be described below.

<Deformation of Air Seal by Gas Pressure>

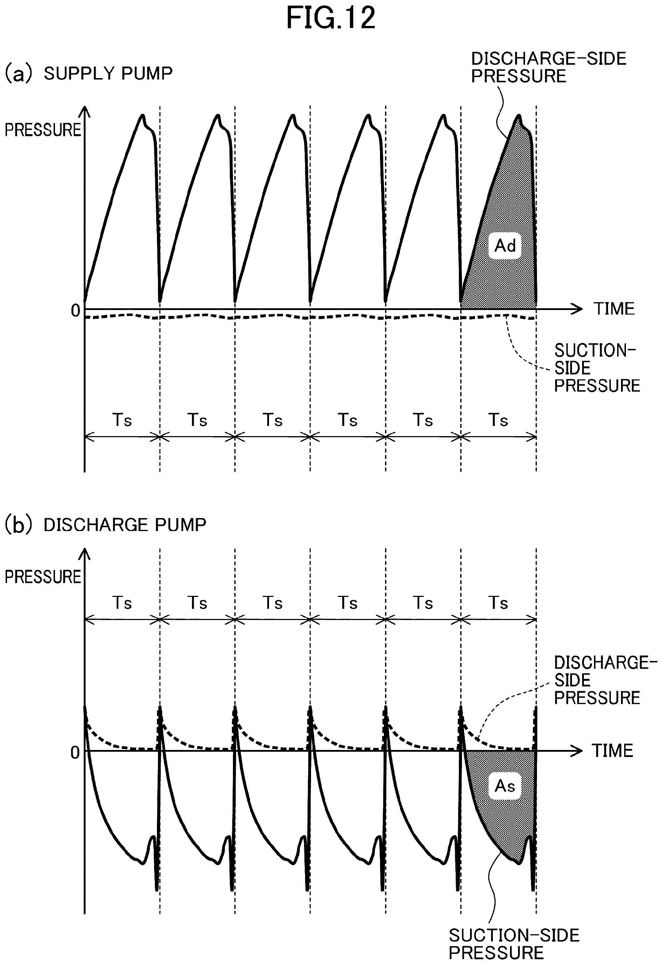

FIG. 12 shows temporal changes in a suction-side pressure and a discharge-side pressure in each of the supply pump (310) and the discharge pump (320). The suction-side pressure is a pressure of gas to be sucked into the gas chamber (317, 327) through the suction port (315a, 325a). The discharge-side pressure is a pressure of gas discharged from the gas chamber (317, 327) through the discharge port (315b, 325b). The suction-side pressure and the discharge-side pressure each periodically fluctuate in conjunction with switching between the first operation and the second operation of the gas composition adjustment apparatus (100).

An average gas pressure acting on the supply-side air seal (313) when the air pump (300) operates can be evaluated by an integral value of the discharge-side pressure shown in FIG. 12. The integral value of the discharge-side pressure of the supply pump (310) is equivalent to the area of a region Ad in FIG. 12. On the other hand, an average gas pressure acting on the discharge-side air seal (323) when the air pump (300) operates can be evaluated by an integral value of the suction-side pressure shown in FIG. 12. The integral value of the suction-side pressure of the discharge pump (320) is equivalent to the area of a region As in FIG. 12. In the air pump (300) of this embodiment, the area of the region Ad related to the supply pump (310) is about 1.5 times the area of the region As related to the discharge pump (320).

In this manner, in the air pump (300) of this embodiment, the average gas pressure acting on the supply-side air seal (313) is higher than the average gas pressure acting on the discharge-side air seal (323). Thus, when the supply-side air seal (313) has the same Young's modulus as that of the discharge-side air seal (323), the supply-side air seal (313) is deformed more due to the gas pressure. As a result, the supply-side air seal (313) is pressed onto the supply-side cylinder (311) with a greater force, and the supply-side air seal (313) is worn more. Thus, desirably, the supply-side air seal (313) has a Young's modulus higher than that of the discharge-side air seal (323).

<Thermal Expansion of Air Seal>

As described above, when the air pump (300) operates, the supply-side air seal (313) has a temperature higher than that of the air discharge-side air seal (323). Thus, when the supply-side air seal (313) has the same linear expansion coefficient as that of the discharge-side air seal (323), the supply-side air seal (313) more expands thermally. As a result, the supply-side air seal (313) is pressed onto the associated supply-side cylinder (311) with a greater force, and the supply-side air seal (313) is worn more. Thus, desirably, the supply-side air seal (313) has a linear expansion coefficient smaller than that of the discharge-side air seal (323).

<Wear Reduction Effect of Carbon Material>

As described above, a fibrous carbon material exhibits a wear reduction effect for an air seal greater than that of a particulate carbon material. However, regarding the supply-side air seal (313) in the air pump of this embodiment, the increase in the amount of wear of the air seal due to "a high average gas pressure acting on the air seal" and "a high temperature of the air seal" exceeds the wear reduction effect of the fibrous carbon material.

To address the problem, the air pump (300) of this embodiment uses the supply-side air seal (313) which is an "air seal containing a fluororesin and a particulate carbon material as main components; having a Young's modulus higher than that of the discharge-side air seal (323); and having a linear expansion coefficient smaller than that of the discharge-side air seal (323)."

-Features of First Embodiment-

In the discharge pump (320) of this embodiment, when the discharge-side air seal (323) slides on the discharge-side cylinder (321), the fibrous carbon material is exposed on the surface of the discharge-side air seal (323) and comes into contact with the discharge-side cylinder (321). The fibrous carbon material has an elongated shape and thus is unlikely to fall off from the discharge-side air seal (323) when coming into contact with the discharge-side cylinder (321). The discharge-side air seal (323) containing a fibrous carbon material as a main component is relatively unlikely to wear because the wear reduction effect of a fibrous carbon material lasts for a long period of time. Thus, in this embodiment, the discharge-side air seal (323) of the discharge pump (320) has a longer life, and the gas composition adjustment apparatus (100) has greater reliability.

In the air pump (300) of this embodiment, the supply-side air seal (313) has a linear expansion coefficient smaller than that of the discharge-side air seal (323). Thus, as compared to the supply-side air seal (313) having the same linear expansion coefficient as that of the discharge-side air seal (323), the supply-side air seal (313) less expands thermally in the supply pump (310) whose temperature becomes higher than that of the discharge pump (320). Thus, in the supply pump (310) of this aspect, the supply-side air seal (313) is pressed onto the supply-side cylinder (311) with a smaller force, and the supply-side air seal (313) has a longer life.

In the air pump (300) of this embodiment, the supply-side air seal (313) has a Young's modulus larger than that of the discharge-side air seal (323). Thus, as compared to the supply-side air seal (313) having the same Young's modulus as that of the discharge-side air seal (323), the supply-side air seal (313) is less deformed by application of a gas pressure in the supply pump (310). Thus, in the supply pump (310) of this aspect, the supply-side air seal (313) is pressed onto the supply-side cylinder (311) with a smaller force, and the supply-side air seal (313) has a longer life.

In this manner, the gas composition adjustment apparatus (100) of this embodiment uses the supply-side air seal (313) which is "an air seal containing a fluororesin and a particulate carbon material as main components;

having a linear expansion coefficient smaller than that of the discharge-side air seal (323); and having a Young's modulus higher than that of the discharge-side air seal (323)," such that the supply-side air seal (313) has a longer life. In other words, the gas composition adjustment apparatus (100) uses the supply-side air seal (313) which is "an air seal having a less wear reduction effect of a carbon material but having a linear expansion coefficient and a Young's modulus each suitable for the supply pump (310)," such that the supply-side air seal (313) has a longer life.

Thus, in the gas composition adjustment apparatus (100) of this embodiment, both the supply-side air seal (313) and the discharge-side air seal (323) have a longer life, and the air pump (300) and the gas composition adjustment apparatus (100) have greater reliability.

-First Variation of First Embodiment-

In the gas composition adjustment apparatus (100) of this embodiment, the controller (110) may have a function of estimating the amount of wear of the air seal (313, 323).

The amount Vw of wear of the air seal (313, 323) is calculated using the following Equation 1.

$$\text{(Equation 1) } Vw = C1 \times F \times L + C2 \times N$$

"C1" is a constant related to the amount of wear of the air seal (313, 323) during operation of the air pump (300). The constant "C1" is determined in advance by conducting a test operation of the air pump (300) when the air pump (300) is designed. The constant "C1" is recorded in the memory device (112) of the controller (110).

"F" is the force with which the air seal (313, 323) is pressed onto the cylinder (311, 321) when the air pump (300) operates. "F" is determined depending on a gas pressure in the gas chamber (317, 327). The gas pressure in the gas chamber (317, 327) during operation of the air pump (300) is determined in advance when the gas composition adjustment apparatus (100) is designed. The force "F" is recorded in the memory device (112) of the controller (110).

"L" is a distance of movement of the air seal (313, 323) in accordance with the reciprocation of the piston (312, 322). The moving distance of the piston (312, 322) for one reciprocating action is determined when the air pump (300) is designed. The number of reciprocating actions of the piston (312, 322) per unit time is also determined when the air pump (300) is designed. Thus, the distance "L" is proportional to the operation time of the air pump (300). Then, the controller (110) calculates the distance "L" based on the operation time of the air pump (300).

"C2" is a constant related to the amount of wear of the air seal (313, 323) caused by activation of the air pump (300). The constant "C2" is determined in advance by conducting a test operation of the air pump (300) when the air pump (300) is designed. The constant "C2" is recorded in the memory device (112) of the controller (110).

"N" is the number of activations of the air pump (300) from a stop state. The controller (110) counts the number of activations of the air pump (300) from a stop state.

The controller (110) records the operation time and the number of activations of the air pump (300). Then, the controller (110) calculates the amount Vw of wear of the air seal (313, 323) using the operation time, the number of activations of the air pump (300), and Equation 1. When the calculated amount Vw of wear of the air seal (313, 323) reaches a predetermined reference value, the controller (110) instructs, e.g., a display panel of the transportation refrigeration apparatus (10) to display a remark to prompt the administrator of the gas composition adjustment apparatus (100) to replace the air seal (313, 323).

-Second Variation of First Embodiment-

The gas composition adjustment apparatus (100) of this embodiment may be installed in a stationary refrigerator or freezer. The gas composition adjustment apparatus (100) of this embodiment may be installed in a refrigerating/freezing container for overland transportation by truck, rail, and the like. The gas composition adjustment apparatus (100) of this embodiment may be installed in a refrigerating/freezing truck including a vehicle body integrated with a box defining a cargo space.

<<Second Embodiment>>

A gas composition adjustment apparatus (100) of this embodiment constitutes an oxygen concentrator. The gas composition adjustment apparatus (100) of this embodiment supplies oxygen-enriched gas to a user such as a patient having a respiratory disease. Here, the differences between the gas composition adjustment apparatus (100) of this embodiment and the gas composition adjustment apparatus (100) of the first embodiment will be described.

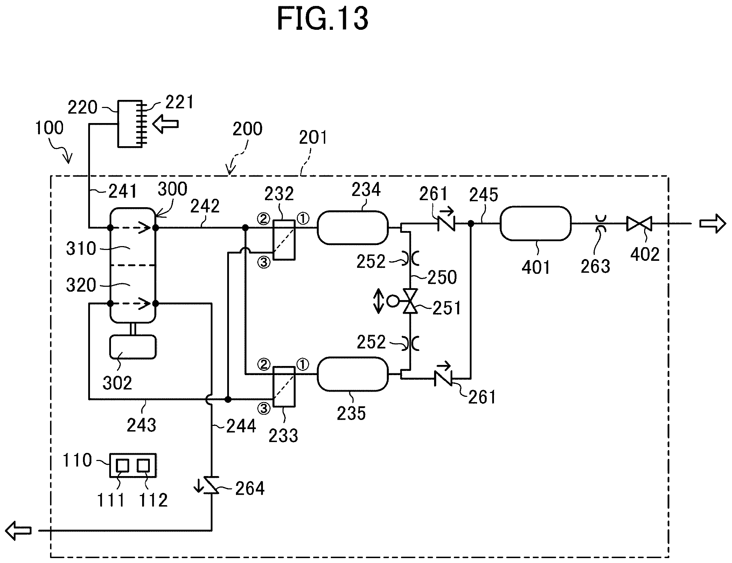

As shown in FIG. 13, in the gas composition adjustment apparatus (100) of this embodiment, a first outflow pipe (244) and a second outflow pipe (245) each extend to the outside of a unit case (201) similarly to the first embodiment. The gas composition adjustment apparatus (100) of this embodiment discharges nitrogen-enriched gas through the first outflow pipe (244) and supplies oxygen-enriched gas through the second outflow pipe (245) to the user.

In the gas composition adjustment apparatus (100) of this embodiment, the second outflow pipe (245) is provided with an oxygen tank (401) and a flow rate control valve (402). The oxygen tank (401) is located upstream of the orifice (263). The opening degree of the flow rate control valve (402) is variable. The flow rate control valve (402) is located downstream of the orifice (263). In the gas composition adjustment apparatus (100) of this embodiment, the ventilation exhaust pipe (150), the sensor unit (160), the bypass connection pipe (255), the exhaust connection pipe (271), and the measurement pipe (281) are omitted.

-Operation of Gas Composition Adjustment Apparatus-

The gas composition adjustment apparatus (100) of this embodiment performs a first operation and a second operation alternately similarly to the gas composition adjustment apparatus (100) of the first embodiment. In principle, the gas composition adjustment apparatus (100) of this embodiment operates continuously without stop.

In the first operation, the oxygen-enriched gas flowing out from the first adsorption vessel (234) is supplied to the user through the second outflow pipe (245). In the first operation, the nitrogen-enriched gas sucked from the second adsorption vessel (235) by the discharge pump (320) is discharged outside through the first outflow pipe (244).

In the second operation, the oxygen-enriched gas flowing out from the second adsorption vessel (235) is supplied to the user through the second outflow pipe (245). In the second operation, the nitrogen-enriched gas sucked from the first adsorption vessel (234) by the discharge pump (320) is discharged outside through the first outflow pipe (244).

While the embodiments and variations thereof have been described above, it will be understood that various changes in form and details may be made without departing from the spirit and scope of the claims. The embodiments and variations thereof may be combined and replaced with each other without deteriorating intended functions of the present disclosure. The ordinal numbers such as "first," "second," ... in the description and claims are used to distinguish the terms to which these expressions are given, and do not limit the number and order of the terms.

INDUSTRIAL APPLICABILITY

As described above, the present disclosure is useful for a gas composition adjustment apparatus.

DESCRIPTION OF REFERENCE CHARACTERS

100    Gas Composition Adjustment Apparatus
234    First Adsorption Vessel (Adsorber)
235    Second Adsorption Vessel (Adsorber)
310    Supply Pump
311    Supply-Side Cylinder (Cylinder)
312    Supply-Side Piston (Piston)
313    Supply-Side Air Seal (Sealing Member)

320    Discharge Pump
321    Discharge-Side Cylinder (Cylinder)
322    Discharge-Side Piston (Piston)
323    Discharge-Side Air Seal (Sealing Member)

**Claims**

1. A gas composition adjustment apparatus (100) comprising:

   an adsorber (234, 235) containing an adsorbent that adsorbs at least one of components of a gas to be treated;
   a supply pump (310) configured to supply the gas to be treated to the adsorber (234, 235); and
   a discharge pump (320) configured to suck a gas from the adsorber (234, 235),
   the gas composition adjustment apparatus (100) being configured to perform

   a pressurization operation in which the supply pump (310) pressurizes the gas to be treated and supplies the gas pressurized to the adsorber (234, 235), and the gas flowing out from the adsorber (234, 235) is sent out as a first gas, and
   a depressurization operation in which the discharge pump (320) sucks a gas from the adsorber (234, 235) and discharges the sucked gas, and the gas discharged from the discharge pump (320) is sent out as a second gas,

   each of the supply pump (310) and the discharge pump (320) being
   a positive displacement pump including a cylinder (311, 321); a piston (312, 322) housed in the cylinder (311, 321); and a sealing member (313, 323) attached to the piston (312, 322) to seal a gap between the cylinder (311, 321) and the piston (312, 322), and
   the sealing member (323) of the discharge pump (320) containing a fluororesin and a fibrous carbon material as main components.

2. The gas composition adjustment apparatus (100) of claim 1, wherein
   the sealing member (313) of the supply pump (310) contains a fluororesin and a particulate carbon material as main components.

3. The gas composition adjustment apparatus (100) of claim 1 or 2, wherein
   the sealing member (313) of the supply pump (310) has a linear expansion coefficient smaller than a linear expansion coefficient of the sealing member (323) of the discharge pump (320).

**4.** The gas composition adjustment apparatus (100) of any one of claims 1 to 3, wherein the sealing member (313) of the supply pump (310) has a Young's modulus higher than a Young's modulus of the sealing member (323) of the discharge pump (320).

**5.** The gas composition adjustment apparatus (100) of any one of claims 1 to 4, wherein

the gas to be treated is atmospheric air,
the adsorbent in the adsorber (234, 235) adsorbs nitrogen which is a component of the gas to be treated,
the first gas has a lower nitrogen concentration and a higher oxygen concentration as compared to the gas to be treated,
the second gas has a higher nitrogen concentration and a lower oxygen concentration as compared to the gas to be treated, and
the gas composition adjustment apparatus (100) is configured to perform an operation of supplying the second gas to a storage (2) for storing a fresh product.

**6.** The gas composition adjustment apparatus (100) of any one of claims 1 to 4, wherein

the gas to be treated is atmospheric air,
the adsorbent in the adsorber adsorbs nitrogen which is a component of the gas to be treated,
the first gas has a lower nitrogen concentration and a higher oxygen concentration as compared to the gas to be treated,
the second gas has a higher nitrogen concentration and a lower oxygen concentration as compared to the gas to be treated, and
the gas composition adjustment apparatus (100) is configured to perform an operation of supplying the first gas to a user.

# FIG.1

# FIG.2

FIG.3

# FIG.4

FIG.5

# FIG.6

# FIG.7

FIG.8

# FIG.9

# FIG.10

# FIG.11

(a) UNWORN

(b) WORN

350

351

350

351

CYLINDER | AIR SEAL

CYLINDER | AIR SEAL

# FIG.12

(a) SUPPLY PUMP

(b) DISCHARGE PUMP

# FIG.13

EP 4 406 638 A1

# EP 4 406 638 A1

## INTERNATIONAL SEARCH REPORT

International application No.

**PCT/JP2022/036902**

### A. CLASSIFICATION OF SUBJECT MATTER

***B01D 53/047***(2006.01)i; ***F04B 27/02***(2006.01)i
FI:   B01D53/047; F04B27/02 D

According to International Patent Classification (IPC) or to both national classification and IPC

### B. FIELDS SEARCHED

Minimum documentation searched (classification system followed by classification symbols)

A01F25/14; A61M16/10; A62B7/00; B01D53/02-053; C01B13/02; C01B21/04; F04B27/02; F04B39/00; F24F8/15

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2016-70608 A (DAIKIN IND., LTD.) 09 May 2016 (2016-05-09)<br>claims, paragraphs [0030]-[0186], fig. 1-4 | 1-2, 5-6 |
| Y | JP 2014-169657 A (IKIKEN KK) 18 September 2014 (2014-09-18)<br>paragraphs [0001], [0015]-[0046] | 1-2, 5-6 |
| Y | JP 2009-85051 A (HITACHI, LTD.) 23 April 2009 (2009-04-23)<br>paragraphs [0002]-[0003], [0017] | 1-2, 5-6 |
| Y | JP 1-262919 A (MITSUI ENG. & SHIPBUILD. CO., LTD.) 19 October 1989 (1989-10-19)<br>fig. 1-2 | 6 |
| A | JP 2013-68111 A (DAIKIN IND., LTD.) 18 April 2013 (2013-04-18) | 1-6 |
| A | JP 2019-66168 A (DAIKIN IND., LTD.) 25 April 2019 (2019-04-25) | 1-6 |

☐ Further documents are listed in the continuation of Box C.    ☑ See patent family annex.

| | |
|---|---|
| * Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **21 November 2022** | **06 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/036902**

| Patent document cited in search report | | | Publication date (day/month/year) | Patent family member(s) | | | Publication date (day/month/year) |
|---|---|---|---|---|---|---|---|
| JP | 2016-70608 | A | 09 May 2016 | (Family: none) | | | |
| JP | 2014-169657 | A | 18 September 2014 | (Family: none) | | | |
| JP | 2009-85051 | A | 23 April 2009 | (Family: none) | | | |
| JP | 1-262919 | A | 19 October 1989 | (Family: none) | | | |
| JP | 2013-68111 | A | 18 April 2013 | US | 2014/0216262 | A1 | |
| | | | | EP | 2759704 | A1 | |
| | | | | CN | 103814214 | A | |
| JP | 2019-66168 | A | 25 April 2019 | WO | 2019/065872 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013042557 A